# EUROPEAN PATENT APPLICATION

(11) **EP 3 178 487 A1**
(43) Date of publication of application: **14.06.2017**
(21) Application number: 15306978.6
(22) Date of filing: 10.12.2015
(51) Int. Cl.: A61K 39/00, C12N 5/0783

(54) **A METHOD FOR OBTAINING HEMATOPOETIC CELL POPULATION CONTAINING REGULATORY T CELLS (TREGS) SPECIFIC FOR AN IRRELEVANT ANTIGEN**

(71) Applicant: INSTITUT NATIONAL DE LA SANTE ET DE LA RECHERCHE MEDICALE (INSERM), 75013 Paris (FR); ASSISTANCE PUBLIQUE-HOPITAUX DE PARIS, 75004 Paris (FR); Université Paris-Est Créteil Val de Marne, 94010 Creteil Cedex (FR); Université Pierre et Marie Curie (Paris 6), 75005 Paris (FR)
(72) Inventor: COHEN, José, 75020 Paris (FR); SALOMON, Benoit, 94100 Saint-Maure (FR); MAURY, Sébastien, 94340 Joinville le Pont (FR); FAUQUIER, Alice, 94500 Champigny sur Marne (FR); THILOAT, Allan, 75020 Paris (FR); SEDDIKI, Nabila, 75015 Paris (FR)
(74) Representative: Regimbeau

(57) **Abstract**

The invention relates to the field of immunology and cell therapy. The invention relates to an *in vitro* or *ex vivo* method for obtaining hematopoietic cell population containing regulatory T cells (Tregs) specific for an irrelevant antigen, to a hematopoietic cell population obtained by said method, to a pharmaceutical composition and a product containing said hematopoietic cell population and therapeutic use thereof.

## Description

### FIELD OF THE INVENTION

The invention relates to the field of immunology and cell therapy. The invention relates to an *in vitro* or *ex vivo* method for obtaining hematopoietic cell population containing regulatory T cells (Tregs) specific for an irrelevant antigen, to a hematopoietic cell population obtained by said method, to a pharmaceutical composition and a product containing said hematopoietic cell population and therapeutic use thereof.

### BACKGROUND OF THE INVENTION

The regulatory T cells (Tregs), also known as suppressor T cells, are a subpopulation of T cells which modulate the immune system, maintain tolerance to self-antigens, and abrogate autoimmune disease. These cells generally suppress or downregulate induction and proliferation of effector T cells (Teffs).

Deficit in Tregs is associated with autoimmune diseases or transplant rejection. For example, graft versus host disease (GVHD), a life threatening complication of allogeneic hematopoietic stem cell transplantation (allo-HSCT) (Ferrara et al., 2009), is an immunological disorder due to effector donor T cells (Teff) present within the graft. Recent clinical trials suggest that adding high numbers of polyclonal regulatory T cells to allo-HSCT reduces GVHD occurrence (Brunstein et al., 2011; Di lanni et al., 2011). It has been observed in mice that the use of specific Treg (rsTreg), specific to the recipient alloantigens, markedly improves this effect, compared to polyclonal Treg (polyTreg) (Cohen et al., 2002; Tranado et al., 2003).

The major drawback of using Tregs specific to an antigen is that it is highly difficult to separate the respective populations of Tregs and Teffs. Hence Teffs are present in the sorted Tregs cell population and thus may aggravate GVHD or induce graft rejection. This problem is increased by the necessity to obtain enough Tregs for the clinical application, as Tregs form a marginal fraction of the circulating CD4⁺ lymphocyte population. It should also be pointed out that most method described in the art rely on flow cytometry for cell separation. However, flow cytometry is not authorized in such context in some countries, including Europe.

Several methods have been implemented allowing Tregs selection. Most common methods use only relevant antigens (i.e. auto-antigens, allo-antigens or allergens involved in the onset of immune diseases caused by pathological T cells) to activate CD4⁺CD25⁺ regulatory T cells in order to favor expansion of Treg preferentially active against pathogenic effector T cells occurring in said immune diseases, such as respectively in autoimmune diseases, graft-rejection, GVHD, allergy, etc.

However, these methods do not lead to a significant reduction of Teffs in Tregs population and do not allow activation on demand of the Tregs.

PCT application WO 2013/076268 discloses a method for obtaining a Treg population containing only low levels of contaminating Teffs which attempts to solve this problem. This method is based on the use of CD4⁺CD25⁺ Tregs specific for an irrelevant antigen. Such Tregs effectively suppress a systemic allogeneic immune response in a robust GVHD mouse model. However, the CD4⁺CD25⁺ Tregs of WO 2013/076268 are sorted by flow cytometry and are thus not easily amenable to clinical utilization. Moreover, this method does not allow obtaining Tregs cell population comprising high numbers of functional, specific Tregs.

Therefore, there is still a need for a method providing functional, specific Tregs with low Teffs contamination, wherein said Tregs can be used in clinical uses.

### DETAILED DESCRIPTION OF THE INVENTION

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the invention, the preferred methods and materials are now described.

As used herein, the term **"PBMC"** or **"peripheral blood mononuclear cells"** refers to any blood cell having a round nucleus (as opposed to a lobed nucleus) such as a lymphocyte, a monocyte or a macrophage. PBMCs are a critical component in the immune system to fight infection and adapt to intruders. The lymphocyte population consists of T cells (CD4⁺ and CD8⁺), B cells and NK cells. These cells can be extracted from whole blood using Ficoll, a hydrophilic polysaccharide that separates layers of blood, which will separate the blood into a top layer of plasma, followed by a layer of PBMCs and a bottom fraction of polymorphonuclear cells (such as neutrophils and eosinophils) and erythrocytes.

**"T cells"** or **"T lymphocytes"** are a type of lymphocyte that plays a central role in cell-mediated immunity. They can be distinguished from other lymphocytes, such as B cells and natural killer cells (NK cells), by the presence of a T-cell receptor (TCR) on the cell surface. A "T cell receptor" or "TCR" as used herein is a receptor present on the surface of T cells that is responsible for recognizing antigens bound to major histocompatibility complex (MHC) molecules. These cells are also known as **CD4⁺ T** cells because they express the CD4 glycoprotein on their surfaces.

As used herein, the term **"regulatory T cells"** or **"Tregs"** refers to population of T cells that express particular cell surface markers, namely CD4 and CD25 markers or CD4⁺CD25^{high}. These cells also express the marker FOXP3 which is a transcription factor (Battaglia & Rancarolo, 2009). Said cells are thus preferably CD4+CD25^{high}FOXP3^{high}. Alternatively, these cells are characterized by the absence of expression of the surface marker CD127 (CD4⁺CD25^{high}CD127). These cells are thus also referred to as natural CD4⁺CD25⁺ regulatory cells. The immunoregulatory CD4⁺CD25⁺ T cells generally represent 3-10% of the normal T-cell compartment in mice and humans. These cells are characterized by an ability to suppress or downregulate immune reactions mediated by effector T cells, such as effector CD4⁺ or CD8⁺ T cells.

As used herein, the term **"exoTregs"** or **"specific Tregs"** refers to Tregs specific to an irrelevant exogenous antigen (i.e., non-donor, non-recipient, antigen), preferably a non-pathogenic antigen or to an exogenous antigen, more preferably to an exogenous non-pathogenic antigen. Preferably, said exoTregs are capable of inhibiting pathogenic Teffs having a different specificity.

The term **"CD4"** as used herein refers to a membrane glycoprotein of T lymphocytes that interacts with major histocompatibility complex class II antigens and is also a receptor for the human immunodeficiency virus. The protein functions to initiate or augment the early phase of T-cell activation. Preferably, the CD4 molecule of the invention is a polypeptide having the amino acid sequence represented by NP_038516.

By **"CD8",** it is herein referred to a cell surface glycoprotein found on most cytotoxic T lymphocytes that mediates efficient cell-cell interactions within the immune system. The CD8 antigen acts as a coreceptor with the T-cell receptor on the T lymphocyte to recognize antigens displayed by an antigen presenting cell in the context of class I MHC molecules. Preferably, the CD8 molecule of the invention is a polypeptide having the amino acid sequence represented by NP_001139345.

The term **"CD25"** as used herein refers to the alpha chain of the IL-2 receptor. This protein is a type I transmembrane protein present on activated T cells, activated B cells, some thymocytes, myeloid precursors, and oligodendrocytes that associates with CD122 to form a heterodimer that can act as a high-affinity receptor for IL-2. Tregs in particular express CD25 in addition to CD4 and FOXP3. Preferably, the CD25 molecule of the invention is a polypeptide having the amino acid sequence represented by NP_032393.

As used herein, the term **"FOXP3"** refers to a transcription factor belonging to the forkhead/winged-helix family of transcriptional regulators. FOXP3 appears to function as a master regulator (transcription factor) in the development and function of regulatory T cells. Preferably, the FOXP3 molecule of the invention is a polypeptide having the amino acid sequence represented by NP_001186276.

The term **"CD127"** herein refers to the interleukin-7 receptor subunit alpha (IL7R-α). The CD127 polypeptide is a type I cytokine receptor which is a subunit of the functional Interleukin-7 receptor and Thymic Stromal Lymphopoietin (TSLP) receptors. Preferably, the CD127 molecule of the invention is a polypeptide having the amino acid sequence represented by NP_002176.

The term **"CD2",** as used herein, refers to a cell adhesion molecule found on the surface of T cells and natural killer (NK) cells. Preferably, the CD2 molecule of the invention is a polypeptide having the amino acid sequence represented by NP_001758.

As used herein, the term **"CD3"** refers to a protein complex composed of four distinct chains, a CD3y chain, a CD3δ chain, and two CD3ε chains. These chains associate with a molecule known as the T-cell receptor (TCR) and the ζ-chain to form together the TCR complex. Preferably, the CD3γ, CD3δ, and CD3ε molecules of the invention are polypeptides having the amino acid sequences represented by NP_000064, NP_000723, and NP_000724, respectively.

As used herein, the term **"antigen"** is any structural substance that serves as a target for the receptors of an adaptive immune response. In particular, an antigen as used herein refers to a predetermined molecule to which a highly variable antigen receptor (B-cell receptor or T-cell receptor) of the adaptive immune system or an antibody can selectively bind. Preferably, antigens are processed and presented to immune cells. Antigen presentation is mediated by MHC class I molecules, and the class II molecules found on the surface of antigen-presenting cells (APCs) and certain other cells. The antigen may be a polypeptide, a carbohydrate, a nucleic acid, a lipid, a hapten or any other naturally occurring or synthetic compound. Preferably, the antigen is a polypeptide. A **"fragment"** of the antigen refers to any subset of the antigen, as a shorter peptide. A **"variant"** of the antigen refers to a molecule substantially similar to either the entire antigen or a fragment thereof. Variant antigens may be conveniently prepared by direct chemical synthesis of the variant peptide, using methods well-known in the art.

As used herein, the terms **"exogenous"** or **"non-allogeneic antigen"** are used herein interchangeably and refer to an antigen which is not expressed by the donor nor by the recipient (also called host within the context of GVHD or organ transplant rejection as well as patient or subject to be treated for other immune diseases). Accordingly, the term refers preferably to an antigen which is not expressed by the species to which the patient to be treated belongs (i.e. including xenogeneic antigen referring to as being from two different species). In other words, if the patient to be treated is a human, the exogenous antigen is not expressed by humans. Moreover, it should be noted that the exogenous antigen is not issued from a microorganism (pathogenic or not such as bacteria, viruses, fungi and parasites) liable to be present in the recipient since, if present, such antigen could re-activate in an unwanted manner and without control the population of Treg specific for said antigen. As used herein, the term "allogeneic" refers to as being from the same species but to as different individuals having two different genetically Major Histocompatibility Complex (MHC) haplotypes. As used herein, the term "syngeneic" refers to genetically identical members of the same species.

As used herein, the term **"irrelevant antigen"** or **"non relevant antigen"** refers to an antigen which is not involved in the disease affecting the patient to be treated. Moreover, the irrelevant antigen may also be a non-pathogenic antigen. As used herein, the term **"pathogenic antigen"** refers to an antigen which is involved in a disease, said disease being different from the disease affecting the patient to be treated. As used herein, the term **"non-pathogenic antigen"** refers to an antigen which is harmless for the patient and/or which is not involved in any disease including non allergic antigen (e.g. not an allergen).

As used herein, an **"antigen presenting cell"** or **"APC"** is an immune cell that mediates the cellular immune response by processing and presenting antigens for recognition by certain lymphocytes such as T cells. Classical APCs include dendritic cells, macrophages, Langerhans cells and B cells.

As used herein, **"effector T cells"** or **"Teffs"** refer to populations of T-cells which secrete cytokines and activate and direct other immune cells. As such, these T cells are the functional cells for executing immune functions (Wan and Flavell, J Mol Cell Biol., 1(1):20-36, 2009).

As used herein, a **"disease caused by pathological T cell"** is a condition which develops as a result of one or more abnormalities in the T-cell system. Such abnormalities include alterations in the number or functional activities of effector or regulatory T cells. These types of reactions may thus lead to the release of cytokines that cause inflammation, cell death and tissue damage. In a first embodiment, a disease caused by pathological T cell is an autoimmune disease such as e.g. SLE, primary biliary cirrhosis, thyroiditis, multiple sclerosis, myasthenia gravis, rheumatoid arthritis and sclerosis. In another embodiment, said disease is graft-versus-host-disease (GVHD).

As used herein, the term **"magnetic activated cell sorting"** refers to a method which allows cells to be separated by incubating said cells with magnetic nanoparticles (or beads) coated with antibodies against a particular surface antigen. This causes the cells expressing this antigen to attach to the magnetic nanoparticles through interaction with the antibodies. Afterwards the cell solution is transferred on a column placed in a strong magnetic field. In this step, the cells attached to the nanoparticles (expressing the antigen) stay on the column, while other cells (not expressing the antigen) flow through. With this method, the cells can be separated positively or negatively with respect to the particular antigen(s).

In positive selection, the cells expressing the antigen(s) of interest, which are attached to the magnetic column, are washed out to a separate vessel, after removing the column from the magnetic field.

In negative selection, the antibody used is against surface antigen(s) which are known to be present on cells that are not of interest. After administration of the cells/magnetic nanoparticles solution onto the column, the cells expressing these antigens bind to the column and the fraction that goes through is collected, since it contains the cells which do not express the undesired antigen(s).

As used herein, the terms **"transplant"** or **"graft"** refer to allogeneic transplants or grafts including, but not limited to whole organs, such as for example, kidney, heart, liver or skin; tissues, such as for example, tissues derived from an organ such as a liver; or cells, such as for example, hematopoietic stem cells. As such an "allograft" is a transplant between two individuals of the same species having two different genetically MHC haplotypes. It should be noted that the term "allograft" encompasses composite tissue allotransplantation (CTA) which is the transfer of a composite tissue that may include skin, muscle, bone and nerve.

As used herein, the terms **"treat", "treating", "treatment",** and the like refer to reducing or ameliorating the symptoms of a disorder (e.g., an immune disease, and/ or symptoms associated therewith). It will be appreciated that, although not precluded, treating a disorder or condition does not require that the disorder, condition or symptoms associated therewith be completely eliminated.

As used herein **"treating"** a disease in a subject or "treating" a subject having a disease refers to subjecting the subject to a pharmaceutical treatment, e.g., the administration of a drug, such that the extent of the disease is decreased or prevented. For examples, treating results in the reduction of at least one sign or symptom of the disease or condition. Treatment includes (but is not limited to) administration of a composition, such as a pharmaceutical composition, and may be performed either prophylactically, or subsequent to, or at the initiation of a pathologic event. Treatment can require administration of an agent and/or treatment more than once.

As used herein, the terms **"patient"** or **"subject"** herein refers to a mammal, preferably a human being.

### An in vitro or ex vivo method for obtaining hematopoietic cell population containing regulatory T cells (Tregs) specific for an irrelevant antigen.

The inventors have found that Tregs specific for an antigen can be isolated from a population of blood cells through immunoselection of at least one Treg-specific marker. This selection enables in particular isolating Tregs from other T cells, such as e.g., conventional T cells (Tconv) or Teffs. It also enables the isolation of the Treg specific of the antigen of interest from the rest of the Tregs. In addition, the inventors have found that amounts of Tregs sufficient for clinical uses may be obtained by expanding the Tregs selected through the immunoselection step.

The invention thus relates to an *in vitro* or *ex vivo* method for obtaining a population of Tregs specific for an irrelevant antigen from a PBMC population, comprising the steps of:
a) contacting said PBMC population with said antigen;
b) isolating Tregs from the PBMCs of step a); and
c) expanding the activated Tregs of step b).

As used herein, a "Treg specific marker" or "Treg marker" refers to any molecule or substance specifically expressed by Treg cells and allowing clearly distinguishing Treg cells from other cell in a cell population.

In the context of the present invention, a cell "expresses a marker" if said marker (e.g. CD4, CD137, CD25, GARP, CD127, CD3, etc.) is present at a significant level in the cell, preferably on the surface of said cell (such a cell being also defined as a "marker⁺ cell"). In particular, a cell expresses said marker if the signal associated to staining the marker (e.g. obtained with an antibody against said marker coupled to a fluorescent marker) which is measured for said cell is similar or identical to the signal corresponding to the same staining of at least one cell being known as expressing said marker. In other terms, the ratio between the signal associated with said marker, measured for said cell and the signal associated with said marker, measured for at least one cell being known as expressing said marker is of about 1. Preferably, the signal associated with said marker of the target cell is compared to an average signal associated with said marker, measured on a population of cells being known as expressing said marker, so that the ratio between the signal associated with said marker, measured for said cell and the average signal associated with said marker, measured on a population of cells being known as expressing said marker is of about 1. Several markers expressed by each blood cell type have been identified, enabling identifying a cell based on the markers it expresses (see e.g., "Human and Mouse CD Marker Handbook" available at www.bdbiosciences.com/documents/cd marker handbook.pdf).

Preferably, a Treg-specific marker is a marker whose expression is restricted to Tregs, said expression being increased after the T-cell receptor (TCR) is engaged. As known in the art, the TCR is a receptor found on the surface of T lymphocytes that is responsible for recognizing antigens bound to major histocompatibility complex (MHC) molecules. The recognition of an antigen by the TCR will trigger lymphocyte activation, which will translate *inter alia* into the expression of a number of surface markers.

The inventors have thus found that selection based on a marker specific of activated Tregs enables the isolation of a hematopoietic cell population highly enriched in functional Tregs from a PBMC sample, with a low level of contamination by other T cells, in particular Tconv and Teff. This population can then be expanded in a second step.

Preferably, the said Tregs have been previously activated by an irrelevant antigen. This activation triggers the expression of said marker specific of activated Tregs, enabling its use in the immunoselection method of the invention. Indeed, the inventors have observed that the proportion of Tregs to Teffs in the population of cells expressing the Tcell-specific marker is even more increased after stimulation by an irrelevant antigen, thus leading to an even more specific selection of Tregs. In addition, Treg specific for an irrelevant antigen effectively suppress allogeneic response. More preferably, said antigen is an exogenous antigen. Exogenous antigens in the context of the present method are of a different species than the Tregs. They are therefore particularly advantageous in the context of the invention, since contaminating Teffs activated by such antigens would have reduced pathogenicity towards the host. Using exogenous antigens thus improve the safety of the method of the invention.

In a first embodiment, said antigen is a non-pathogenic antigen. According to one embodiment, the irrelevant non-pathogenic antigen of the present invention is an immunogenic peptide that can bind to MHC class II molecule of an individual, and that is recognized by the T cell receptor of said individual, while being harmless for said individual. For example, the irrelevant non-pathogenic exogenous antigen may be a food antigen from common human diet, preferably a non-allergic food antigen for the patient to be treated.

As used herein, the term "food antigen from common human diet" refers to an immunogenic peptide, which comes from foodstuff common for humans, such as food antigens of the following non-limiting list: bovine antigens such as lipocalin, Ca-binding SI00, alpha- lactalbumin, lacto globulins such as beta- lacto globulin, bovine serum albumin, caseins. Food antigens may also be Atlantic salmon antigens such as parvalbumin, chicken antigens such as ovomucoid, ovalbumin, Ag22, conalbumin, lysozyme or chicken serum albumin, peanuts, shrimp antigens such as tropomyosin, wheat antigens such as agglutinin or gliadin, celery antigens such as celery profilin, carrot antigens such as carrot profilin, apple antigens such as thaumatin, apple lipid transfer protein, apple profilin, pear antigens such as pear profilin, isoflavone reductase, avocado antigens such as endochitinase, apricot antigens such as apricot lipid transfer protein, peach antigens such as peach lipid transfer protein or peach profilin, soybean antigens such as HPS, soybean profilin or (SAM22) PR-10 prot.

Alternately, in another embodiment, an irrelevant pathogenic antigen may be used in the method of the invention. Such an antigen is an antigen which is not involved in the disease to be treated but may be involved in another disease (It should be further noted that the patient is not affected with said other disease). Indeed, depending the disease to be treated, irrelevant pathogenic antigen may also be used. For instance, an allergen is involved in allergy (and is a pathogenic antigen) but it is also an irrelevant antigen for other diseases that allergy. Therefore, an allergen is not involved in GVHD, organ transplant rejection or auto-immune diseases if the pathology to be treated is selected group consisting of GVHD, organ transplant rejection or auto-immune diseases. Irrelevant pathogenic antigens thus encompass allo-antigens or allergens involved in the onset of immune diseases caused by pathological T cells.

More preferably, the irrelevant antigen is Keyhole Limpet Hemocyanin or KLH, a non-pathogenic exogenous antigen. KLH is a naturally occurring immunoadjuvant functioning as a respiratory protein of giant keyhole limpets *Megathura crenulata* living in shallow coastal waters in a sea. It is a mixture of two immunologically distinct isoforms, both of which are didecamers assembled from 400 kDa polypeptides; an atomistic model of the quaternary structure of isoform KLH1 is available (C. Gatsogiannis and J. Markl, "Keyhole limpet hemocyanin: 9-Å CryoEM structure and molecular model of the KLH1 didecamer reveal the interfaces and intricate topology of the 160 functional units," Journal of Mol. Biol. 385(3): 963-983, 2009).

This antigen is ordinarily not encountered by the human immune system. However, its large size and numerous epitopes generate a substantial immune response. At very low concentration this antigen is capable to induce an *in vitro* or *in vivo* T dependent immune response.In addition, because KLH is derived from the limpet, a gastropod, it is phylogenetically distant from mammalian proteins. Indeed, KLH has been widely used as a protein carrier to numerous poorly immunogenic cancer antigens such as in vaccines against follicular lymphoma, non-Hodgkin lymphoma, glioblastoma multiforme, melanoma, prostate, and ovarian cancer. The conjugate cancer vaccines appear as the most extensively studied application of KLH at the moment.

In a particular embodiment, the antigen is presented by an antigen-presenting cell ("APC"). According to this embodiment, "contacting a PBMC population with an irrelevant antigen" refers to the presentation of said antigen by APCs. APCs are preferably dendritic cells.The APCs may be cells isolated from the donor or from the patient. They may be selected to produce activated immunoregulatory T cells having a desired activity profile. Method for isolating functional APCs from PBMC are well known in the art. Advantageously, the APCs of the invention are obtained by negative selection for a specific marker. Negative selection can be performed by any means known to the person of skills in the art. Preferably, negative selection is performed by incubating the cells with an antibody directed against said specific marker and retaining those cells which are not bound by said antibody. In a preferred embodiment, said marker is CD3 and the APCs are CD3⁻ cells. In another preferred embodiment, said marker is CD2. According to this embodiment, the APCs are CD2⁻ cells.

In order to limit the contamination of the Tregs population by the APCs, said APCs may be irradiated, for example at 30 grays. Irradiated APCs rapidly die (the inventors have found that 90 % of them are dead 7 days after irradiation), which decreases the risk of contaminating the population of Tregs obtained by the method of the invention. In addition, the inventors have shown that irradiation does not impact the Treg stimulation by the irrelevant antigen presented by said irradiated APCs. Accordingly, in a preferred embodiment of the method of the invention, the APCs have been previously irradiated.

It should be further noted that the APCs may be autologous or allogeneic to regulatory T cells or may be artificial APCs.

In this regard, in the particular case of treating graft-versus-host-disease (GVHD), donor-type immunoregulatory T cells are preferably selected and activated by APCs isolated from the same donor prior to the hematopoietic stem cell transplantation (HSCT).

On the other hand, for treating allografts, immunoregulatory T cells are typically isolated from the patient (or recipient) and activated by APCs from the patient.

Finally, for treating autoimmune diseases or allergies, immunoregulatory T cells are preferably isolated from the patient and activated by autologous APCs.

The present invention thus relates to an *in vitro* or *ex vivo* method for obtaining a population of Tregs specific for an irrelevant antigen from a PBMC population, comprising the steps of:
a) contacting said PBMC population with said antigen;
b) selecting the cells expressing a marker specific of activated Tregs from the population obtained in step a); and
c) expanding the activated Tregs of step b).

The PBMC population used in the method of the invention may be obtained from any type of biological sample known to contain PBMC. As used herein, the term "biological sample" or "sample" refers to a whole organism or a subset of its tissues, cells or component parts. "Biological sample" further refers to a homogenate, lysate or extract prepared from a whole organism or a subset of its tissues, cells or component parts, or a fraction or portion thereof. The biological sample to be measured by the test method of the present invention is not particularly limited, as far as it can be collected from a mammal, preferably from a human; examples include humoral samples such as blood, bone marrow fluid, and lymph fluid, and solid samples such as lymph nodes, blood vessels, bone marrow, brain, spleen, and skin. Preferably, a "biological sample" according to the invention is any tissue which may contain PBMC, e.g., blood, plasma, or bone marrow.

Since PBMC are mostly found in the blood, it is particularly advantageous to use blood as a biological sample for the method of the invention. Indeed, such a blood sample may be obtained by a completely harmless blood collection from the subject.

Several methods for selecting cells expressing a specific marker are available to the person of skills in the art. Conventional methods for cell isolation which enrich subpopulations of cell mixtures include, e.g., density gradient separation, fluorescence activated cell sorting, immunological cell separation techniques such as panning, complement lysis, resetting, magnetic cell separation techniques, and nylon wool separation. Different patterns of expression of cell surface antigens have been used in some cases to identify different cell types. Certain disadvantages of many of these reported methods are that they can be time-consuming, labor-intensive, costly, require large amounts of reagent, result in low specificity, low sensitivity, contaminated mixtures, poor and/or inaccurate separation, and the loss of desired cells. Certain other methods, such as the treatment of the cell population with cytokines, can change the properties, functions, or viability of the desired cells. Thus, prior methods generally are inefficient, time-consuming, expensive, and do not optimize for pure populations. Moreover, these methods are not suitable for obtaining clinical-grade populations of Tregs. In particular, the use of flow cytometry is not appropriate for selecting cells which are going to be used in clinical conditions.

The inventors have discovered that magnetic activated cell sorting (MACS) provides an improved method of cell separation over techniques employed in the prior art in the context of the present invention. MACS is versatile and innocuous to the cells (Miltenyi, S. et al., Cytometry.11:231- 238(1990)). MACS has found many useful applications in immunological research, especially in separating lymphocyte subsets based on their expression of cell surface antigens. The technique involves coupling the cell surface with biodegradable microbeads the size of cellular macromolecules (50-150 nm diameter). In a preferred embodiment, the microbeads are attached to antibodies specific for a surface antigen expressed by the target cell. According to this embodiment, the microbeads become coupled to the cell surface when the antibody binds the target antigen. The cells are then passed through a magnetizable matrix in a strong magnetic field. Labeled cells stick to the matrix and are separated from unlabeled cells, which flow through. The retained antibody-cell complex can then be eluted from the matrix, e.g., by demagnetizing the matrix, e.g., by removing the matrix from the magnetic field. Devices for practicing the technique are commercially available (see e.g., Miltenyi Biotec, Bergisch Gladbach, Germany).

Preferably, the cells expressing a specific marker, particularly Tregs, are isolated by magnetic activated cell sorting. More preferably, said isolation involves contacting said cells with an antibody recognizing said specific marker, wherein said antibody is attached to microbeads. When the selection is positive, the cells bound by the antibody are retained; when the selection is negative, the cells bound by the antibody are discarded.

The term "antibody" as used herein is intended to include monoclonal antibodies, polyclonal antibodies, and chimeric antibodies. More particularly, an antibody (or "immunoglobulin") consists of a glycoprotein comprising at least two heavy (H) chains and two light (L) chains inter-connected by disulfide bonds.

A "monoclonal antibody", as used herein, means an antibody arising from a nearly homogeneous antibody population. More particularly, the individual antibodies of a population are identical except for a few possible naturally-occurring mutations which can be found in minimal proportions. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one class and subclass, and light chains of only one type. Monoclonal antibodies are highly specific and are directed against a single antigen. In addition, in contrast with preparations of polyclonal antibodies which typically include various antibodies directed against various determinants, or epitopes, each monoclonal antibody is directed against a single epitope of the antigen.

The antibody may be from recombinant sources and/or produced in transgenic animals. Conventional techniques of molecular biology, microbiology and recombinant DNA techniques are within the skill of the art. Such techniques are explained fully in the literature.

To produce monoclonal antibodies, antibody producing cells (lymphocytes) can be harvested from an immunized animal and fused with myeloma cells by standard somatic cell fusion procedures thus immortalizing these *cells* and yielding hybridoma cells. Such techniques are well known in the art (e. g. the hybridoma technique originally developed by Kohler and Milstein (Nature, 256: 495-497,1975) as well as other techniques such as the human B-cell hybridoma technique (Kozbor et al, Immunol Today, 4: 72-79, 1983), the EBV-hybridoma technique to produce human monoclonal antibodies (Cole et al., Methods Enzymol, 121: 140-167,1986), and screening of combinatorial antibody libraries (Huse et al., Science, 246: 1275-1281, 1989) Hybridoma cells can be screened immunochemically for production of antibodies specifically reactive with the polypeptide and the monoclonal antibodies can be isolated.

Antibodies may be isolated after production (e.g., from the blood or serum of the animals) or synthetized and further purified by well-known techniques. Antibodies specific for a protein can be selected or purified by affinity chromatography, ELISPOT or ELISA. For example, a recombinantly expressed and purified (or partially purified) marker of interest may be produced, and covalently or non-covalently coupled to a solid support such as, for example, a chromatography column. The column can then be used to purify antibodies specific for the biomarkers of the invention from a sample containing antibodies directed against a large number of different epitopes, thereby generating a substantially purified antibody composition, i.e., one that is substantially free of contaminating antibodies. By a substantially purified antibody composition it is meant, in this context, that the antibody sample contains at most only 30% (by dry weight) of contaminating antibodies directed against epitopes other than those of the biomarkers of the invention, and preferably at most 20%, yet more preferably at most 10% and most preferably at most 5% (by dry weight) of the sample is contaminating antibodies. A purified antibody composition means that at least 99% of the antibodies in the composition are directed against the desired biomarkers of the invention.

Several markers specifically expressed in activated Tregs may be used in the selection step of the methods of the invention. The inventors have shown that two markers, CD137 and GARP, are especially suited for the purposes of the method.

Preferably, in the method of the present invention, the population of Tregs specific for irrelevant antigen is selected via CD137 or GARP. These markers allow not only specifically distinguishing Tregs from other cells of the cell population, including Tconvs and Teffs, especially Teffs, but also distinguishing Tregs specific to an irrelevant antigen of interest from the rest of the Tregs.

CD137 (4-1BB), is an inducible T-cell costimulatory receptor and a member of the tumor necrosis factor receptor (TNFR) superfamily. CD137 expression is induced in T cells after activation. Human CD137 preferably has the amino acid sequence represented by NP_001552.

GARP (glycoprotein-A repetitions predominant; LRRC32) is a type I membrane protein with twenty-two leucine-rich repeats and a short intracytoplasmic region, which is specifically induced in CD4⁺ CD25^{high}FOXP3^{high} Treg cells and thus is a Treg-specific activation marker. Activated Tregs co-express latent TGF-B and GARP on their surfaces, to which GARP is critical to tether TGF-B. GARP thus participates in the TFG-B secretion which is involved in suppressor function of Tregs. Human GARP preferably has the amino acid sequence represented by NP_001122394.

In a preferred embodiment of the method of the invention, the marker specific for activated Tregs is CD137 or GARP. According to this embodiment, the method of the invention comprises the steps of:
a) contacting said PBMC population with an irrelevant antigen;
b) selecting the cells expressing a marker specific of activated Tregs from the population obtained in step a), said marker being selected from the group consisting of CD137 and GARP); and
c) expanding the activated Tregs of step b).

Advantageously, the method of the present invention comprises a prior step of isolating all T cells (i.e., Treg, Tconv and Teff) from the PBMC population, thus further enriching the population of Tregs. In subsequent steps, the isolated T cells are contacted with an irrelevant antigen, followed by the selection of the cells expressing a marker specific of activated Tregs from the population contacted with said antigen, said marker being selected from the group consisting of CD137 and GARP.

Preferably, since T cells are known to express CD4, the prior isolation of all T cells is performed by selecting CD4⁺ cells. Indeed, the inventors have shown that preselecting CD4⁺ is particularly advantageous since the resulting yield of activated Tregs is increased almost two fold.

The common method for Treg cell sorting performed by microbeads coated with antibodies against CD4⁺ and CD25⁺ (positive selection) do not lead to a sufficient increase of Tregs number.

The person of skills in the art will easily realize that MACS only allows one step of positive selection. Once the Tregs of interest are bound specifically by an antibody-microbead complex, they cannot be eluted from this complex without being submitted to a potentially-damaging procedure. Thus, in a preferred embodiment, the selection of CD4⁺ cells is a negative selection, i.e. CD4⁺ cells are selected by contacting the PBMC population with antibodies targeting markers not present on CD4⁺ cells, i.e., T cells, and retaining the cells not bound by said antibodies. For example, non-CD4⁺ cells, i.e., CD8⁺ T cells, monocytes, neutrophils, eosinophils, B cells, dendritic cells, NK cells, granulocytes, y/δ T cells, and erythroid cells are bound by antibodies recognizing specifically each of these cell types. Lists of markers specifically expressed by any one of these cell types are well known in the field; some are available from commercial suppliers of laboratory tools (see e.g., "Human and Mouse CD Marker Handbook" from Becton Dickson; available atwww.bdbiosciences.com/documents/cd_marker_handbook.pdf). According to a preferred embodiment, CD4+ cells are selected by contacting the PBMC with antibodies directed against CD14 (nip_033971), CD8, CD19 (NP 033974), CD36 (NP_001153027), CD56 (NP_001074914), CD123 (NP_032395), and TCR y/δ and only the cells not bound by these antibodies are retained. According to a further preferred embodiment, the PBMC are contacted with antibodies directed against CD14 (NP_033971), CD8, CD19 (NP 033974), CD36 (NP 001153027), CD56 (NP _001074914), CD123 (NP_032395), TCR y/δ, and CD127, and only the cells not bound by these antibodies are retained for the next steps of being contacted with an irrelevant antigen, and selecting the cells expressing a marker specific of activated Tregs from the population contacted with said antigen, said marker being selected from the group consisting of CD137 and GARP.

The results of the inventors show that such a prior enrichment of CD4⁺ cells by negative selection leads to decrease in the Teff contamination and an increase in the frequency of Tregs.

Thus, in a more preferred embodiment, the invention relates to an *in vitro* or *ex vivo* method for obtaining a population of Tregs specific for an irrelevant antigen from a PBMC population, comprising the steps of:
a) obtaining CD4⁺ cells from said PBMC population by negative selection;
b) contacting the CD4⁺ cells of step a) with an irrelevant antigen;
c) selecting the cells expressing a marker specific of activated Tregs from the population obtained in step b), said marker being selected from the group consisting of CD137 and GARP); and
d) expanding the activated Tregs of step c).

In a preferred embodiment, step a) of the method of the invention comprises the steps of:
i) adding antibodies directed against-CD14, CD8, CD19, CD36, CD56, CD123, and TCRγ/∂ to said PBMC population; and
ii) recovering cells not bound by any antibody in step i).

In a more preferred embodiment, an antibody directed against CD127 is also contacted with the cells and the cells not bound by said anti-CD127 antibody are recovered. The anti-CD127 antibody can be added directly with the antibodies of step i) or it can be added to the cells recovered in step ii). In the latter case, step a) comprises the further steps of:
iii) adding an antibody directed against CD127 to the cells recovered in step ii); and
iv) recovering the cells not bound by the antibody in step iii).

In order to obtain a sufficient amount of Tregs, the expansion step is a critical element for clinical applications. Tregs represent only about 5 % of the original CD4⁺ cell population. As used herein the terms "expanding" and "expansion" refer to substantially differentiation-less maintenance of the cells and ultimately cell growth, i.e., increase of a cell population (e.g., at least 2 fold) without differentiation accompanying such increase. The expansion step of the method of the invention thus encompasses a phase of growth and multiplication of the Tregs obtained through the previous immunoselection procedure. Preferably, said Tregs will be expanded in a growth medium and under conditions enabling such cells to grow and multiply. Such medium and conditions are well known in the art (see e.g., Raulf-Heimsoth, T cell - primary culture from peripheral blood, 2008, Methods Mol Med., 138: 17-30) and may be available from commercial suppliers (see e.g., " T Cell Activation/Expansion Kit", Miltenyi Biotec, Bergisch Gladbach, Germany).

The expansion step aims at obtaining cell numbers sufficient for clinical use such as cellular therapy. In addition, this phase can be advantageously coupled with at least one round of stimulation/activation of the Tregs in order to further increase the antigen specificity of said Tregs. Stimulating/activating the Tregs has been shown to result in a direct increase of the number of specific, activated Tregs, by comparison with an expansion lacking any such stimulation/activation phase.

According to this embodiment, the Tregs are grown in the presence of APC presenting the irrelevant antigen used in the immunoselection. Advantageously, the Tregs are grown in the presence of the irrelevant antigen-presenting-APC for at least 2, more preferably at least 3, still more preferably at least 4, even more preferably at least 5 generation time. The "generation time" as used herein is the period of time it takes for a cell of a particular cell type, e.g., a Treg, to divide into two daughter cells. Generation times of each particular cell type are usually well known in the field, but can also be easily determined by the skilled person. Stimulating the Treg population with the irrelevant antigen during 2 or 3 generation times increases the antigenic specificity of the Treg population.

The APCs are preferably dendritic cells, In a further preferred embodiment, the APCs are CD3⁻ cells. In another further preferred embodiment, said marker is CD2. According to this embodiment, the APCs are CD2⁻ cells. In a more preferred embodiment, the APCs are irradiated, for example at 30 grays, in order to prevent the dilution of the population of Tregs obtained by the method of the invention.

Any molecule known to promote expansion of Tregs may be added in the growth medium during the expansion step. In particular, interleukin 2 (IL-2) is a major cytokine involved in regulating lymphocyte proliferation. It is known that the cytokine IL-2 promotes Tregs expansion (see e.g., Elpek et al., Ex vivo expansion of CD4+CD25+FoxP3+ T regulatory cells based on synergy between IL-2 and 4-1BB signaling. J Immunol. 179(11):7295-7304, 2007). Il-2 is preferably added in the growth medium in any amount known to promote Treg expansion. For example, IL-2 is added at a concentration of 100 IU/ml.

In addition, it may be advantageous to add rapamycin (also known as sirolimus) in the growth medium, since rapamycin is known to inhibit Teffs whilst promoting Treg expansion (see e.g., Strauss et al., Differential responses of human regulatory T cells (Treg) and effector T cells to rapamycin, 2009, PLoS One. 4(6): e5994). Rapamycin is commercially available, e.g., it is currently marketed under the trade name Rapamune by Pfizer). The amount of rapamycin required for decreasing Teff number in the method of the invention can easily be determined by the skilled artisan. For example, rapamycin may be added at around 100 nM in the growth medium. Any other compound specifically inhibiting Teffs but not Tregs may be used in the method of the invention.

Advantageously, the steps of contacting the Tregs with the irrelevant-antigen presenting-APC and isolating the resulting activated Tregs by negative selection are performed at least 2, more preferably at least 3, still more preferably at least 4, even more preferably at least 5 times. Performing several cycles of antigen stimulation allows obtaining Tregs cells population containing increased number of Tregs having high antigenic specificity and very low number of undesirable Teffs. Such cell population is suitable for clinical use.

### Hematopoietic population containing regulatory T cells specific for an irrelevant antigen obtained according to the method of the invention and pharmaceutical compositions thereof

In another aspect, the present invention also relates to the hematopoietic cell population containing regulatory T cells (Tregs) specific to irrelevant antigen as defined above, obtained by the method described above.

The hematopoietic cell population according to the present invention contains an increased number of Tregs having improved suppressor functionality and improved specificity to an irrelevant antigen. Moreover, the number of Teffs is significantly reduced. Consequently, this hematopoietic cell population is suitable to clinical use. The hematopoietic cell population as obtained by the method of the invention contains at least 80-90% of Tregs, preferably Tregs CD4⁺ cells with antigenic specificity >80% for the irrelevant antigen used, e.g., the KLH antigen.

According to one embodiment of the present invention, the Tregs in said hematopoietic cell population are selected from the group consisting of: CD4⁺CD25⁺, CD4⁺CD25^{high} and CD4⁺CD25 ^{high}FOXP3+^{high}.

According to an aspect, the present invention also provides a pharmaceutical composition comprising at least one hematopoietic population of regulatory T cells as defined above. The pharmaceutical composition may generally include one or more pharmaceutically acceptable and/or approved carriers, additives, antibiotics, preservatives, adjuvants, diluents and/or stabilizers. Such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycollic acids, polymeric amino acids, amino acid copolymers, lipid aggregates, or the like. This pharmaceutical composition can contain additional additives such as mannitol, dextran, sugar, glycine, lactose or polyvinylpyrrolidone or other additives such as antioxidants or inert gas, stabilizers or recombinant proteins (e. g. human serum albumin) suitable for in vivo administration.

As used herein, the term "pharmaceutically acceptable" refers to molecular entities and compositions that do not produce an adverse, allergic or other untoward reaction when administered to a mammal, especially a human, as appropriate. As used herein, "pharmaceutically acceptable carrier" includes any and all solvents, buffers, salt solutions, dispersion media, coatings, antibacterial and antifungal agents, isotonic and absorption delaying agents, and the like that are physiologically compatible. The type of carrier can be selected based upon the intended route of administration. In various embodiments, the carrier is suitable for intravenous, intraperitoneal, subcutaneous, intramuscular, topical, transdermal or oral administration. Pharmaceutically acceptable carriers include sterile aqueous solutions or dispersions and sterile powders for the extemporaneous preparation of sterile injectable solutions or dispersion. The use of media and agents for pharmaceutically active substances is well known in the art. As detailed herebelow, additional active compounds can also be incorporated into the compositions, such as anti-cancer and/or anti-angiogenesis agents; in particular, the additional active compound can be an anti-angiogenic agent, a chemotherapeutic agent, or a low-molecular weight agent. A typical pharmaceutical composition for intravenous infusion could be made up to contain 250 ml of sterile Ringer's solution, and 100 mg of the combination. Actual methods for preparing parenterally administrable compounds will be known or apparent to those skilled in the art and are described in more detail in for example, Remington's Pharmaceutical Science, 17th ed., Mack Publishing Company, Easton, Pa. (1985), and the 18th and 19th editions thereof, which are incorporated herein by reference.

### Therapeutic methods and uses

Another aspect of the present invention relates to a population of Tregs obtainable by a method as defined above for use as a drug.

More particularly, the present invention relates to a population of Tregs obtainable by a method as defined above or a pharmaceutical composition comprising thereof for use in the prevention or treatment of immune diseases.

As previously mentioned, the invention is suited for preventing or treating immune diseases, such as various diseases caused by pathological T cells, including graft-versus-host- disease (GVDH), autoimmune diseases, graft rejection, allergies, etc.

Accordingly, in one embodiment the population of Tregs may be specific for an irrelevant non-pathogenic exogenous antigen as defined above. In a preferred embodiment, said irrelevant antigen is KLH.

In a particular embodiment, the population of Tregs is a population of Tregs specific for a food antigen from a common human diet.

In a preferred embodiment, the population of interest is a population of Tregs specific for ovalbumin (also called ovaTreg).

Alternatively, in another embodiment the population of Tregs may be specific for an irrelevant pathogenic antigen (depending to the disease to be treated).

Thus, another aspect of the present invention also relates to a method of treating or preventing an immune disease in a patient in need thereof comprising the following steps of:
a) obtaining in vitro or ex vivo a population of Tregs specific for an antigen which is not involved in the immune disease to be treated, by any of the methods described above; and
b) administering to said patient in need thereof the population of step a).

In a particular embodiment, the method further comprises administering the antigen used in step a) to the patient. This administration aims at maintaining the Tregs activated and suppression active, thus reducing the risks for the patient. Said administration can be performed simultaneously, separately or sequentially by direct administration of the antigen to the patient.

More specifically, the antigen may be administered at the step c) to the patient simultaneously, separately or sequentially by administration of antigen presenting cells (APCs) pulsed with the antigen of interest to the patient as previously described. APCs pulsed with the antigen of interest may be conveniently prepared by methods well-known in the art. In a preferred embodiment, the APCs are dendritic cells. In a further preferred embodiment, the APCs are CD3⁻ cells. In another further preferred embodiment, said marker is CD2. According to this embodiment, the APCs are CD2⁻ cells. In a more preferred embodiment, the APCs are irradiated, for example at 30 grays.

In another preferred embodiment, the APCs are pulsed with a food antigen from common human diet such as ovalbumin, fragments and variants thereof (ovapeptide).

In yet another preferred embodiment, said APCs are pulsed with KLH.

It should be further noted that the APCs may be autologous or allogeneic to Tregs or may be artificial APCs.

Therefore, it is particularly suited for the prevention or treatment of GVHD in a subject undergoing allogeneic organ transplantation. By "graft versus host disease (GVHD)" or "GVHD", it is herein referred to an immune-mediated disease resulting from a complex interaction between donor and recipient adaptive immunity following an allogeneic tissue transplant. GVHD can result for example from organ transplantation, such as e.g., kidney transplantation. Most frequently, GVHD occurs after allogeneic bone marrow or hematopoietic stem cell transplantation. Allogeneic bone marrow or hematopoietic stem cells are frequently transplanted in cancer patients after chemotherapy or radiotherapy, since such treatments kill endogenous stem cells present in the patient's bone marrow. For instance, hematopoietic stem cells may be transplanted to a recipient suffering from a hematological malignant disease, including leukemia such as acute lymphoblastic leukemia (ALL), acute nonlymphoblastic leukemia (ANLL), acute myelocytic leukemia (AML) and chronic myelocytic leukemia (CML).

The present application demonstrates that ex vivo expanded Tregs, which have been activated by an irrelevant antigen, can also control GVHD, whether said population is subsequently re-activated by an administration of said antigen to the patient to be treated.

In this regard, the invention relates to a method of preventing or treating GVHD in a subject undergoing HSC or bone marrow transplantation, the method comprising administering to the subject, prior to, during or after HSC or bone marrow transplantation, an amount of immunoregulatory T cells specific for an antigen according to the invention effective at preventing or treating GVHD in said subject and then administering to said patient simultaneously, separately or sequentially the antigen used to previously activate in vitro or ex vivo the population of Tregs.

In preferred embodiments, the cells are ex vivo expanded and/or administered together with transplantation, optionally followed by subsequent administration(s) depending on the appearance of delayed clinical signs of GVHD.

Accordingly, in particular embodiment, the method of preventing or treating GVHD in a patient undergoing HSC or bone marrow transplantation from a transplant obtained from a donor comprises the following steps of:
a) isolating a population of Tregs from the donor;
b) obtaining in vitro or ex vivo a population of Tregs specific for an antigen which is not involved in the GVHD to be treated, by any of the methods described above;
c) administering to said patient in need thereof the population of step b).

In a particular embodiment, the method further comprises administering the antigen used in step b) to the patient. Said administration can be performed simultaneously, separately or sequentially by direct administration of the antigen to the patient.

In one embodiment, the patient undergoing HSC or bone marrow transplantation is a patient affected with a hematological malignant disease

In one embodiment, the step c and/or the step of administering the antigen is(are) carried out simultaneously, separately or sequentially to the step of allogeneic organ transplantation, for example allogeneic bone marrow or hematopoietic stem cell transplantation. In a particular embodiment, the antigen is selected from the group consisting of irrelevant non-pathogenic exogenous antigens (e.g. a food antigen from a common human diet including ovalbumin, fragments and variants thereof or a protein from a non-related organism such as e.g., KLH) and irrelevant pathogenic exogenous antigens (e.g., allergens).

It should further noted that in the particular case of treating graft-versus-host-disease (GVHD), donor-type immunoregulatory T cells are preferably used and activated by APCs isolated from the same donor prior to the transplantation.

The invention is also suited for the prevention or treatment of autoimmune diseases (including chronic inflammatory diseases), such as systemic lupus erythematosus, rheumatoid arthritis, polymyositis, multiple sclerosis, diabetes, etc. Autoimmune diseases have a clear immunological component, as shown by various biological and histological investigations. For these diseases, the central element is an unsuitable immune response. Furthermore, in these diseases it is generally possible to identify the auto-antigen and to define the period of time during which the deleterious Teffs are activated. The present invention can be used to treat, reduce or alleviate such diseases by administering to the subject an effective amount of Tregs specific for an antigen according to the invention effective to suppress or reduce the activity of such deleterious Teffs. Repeated administrations may be contemplated, if needed.

Accordingly, in particular embodiment, the method of preventing or treating an autoimmune disease in a patient in need thereof comprises the following steps of:
a) isolating a population of Tregs from the patient;
b) obtaining in vitro or ex vivo a population of Tregs specific for an antigen which is not involved in the immune disease to be treated, by any of the methods described above;
c) administering to said patient in need thereof the population of step b).

In a particular embodiment, the method further comprises administering the antigen used in step b) to the patient. Said administration can be performed simultaneously, separately or sequentially by direct administration of the antigen to the patient.

In a particular embodiment, the antigen is selected from the group consisting of irrelevant non-pathogenic exogenous antigens (e.g. a food antigen from a common human diet including ovalbumin, fragments and variants thereof, or a protein from a non-related organism such as e.g., KLH) and irrelevant pathogenic antigens (e.g. allergens and allo antigens).

It should further be noted that for treating autoimmune diseases, Tregs are preferably isolated from the patient and activated by autologous APCs.

The present invention can also be used for the prevention or the treatment of organ transplant rejection, such as heart, liver, cornea, kidney, lung, pancreas, etc. The conventional treatment for a certain number of organ disorders is, when it becomes necessary, replacement of this organ with a healthy organ originating from a dead donor (or a living donor in certain cases). This is also the case for treating certain insulin-dependent diabetes, through the grafting of insulin-producing cells or organs, such as pancreas or pancreatic islets. While rigorous care is taken in selecting the organ donors with the maximum compatibility vis-a-vis the MHC antigens, apart from transplants between homozygotic twins, the organ transplant always leads to the development of an immune response directed against the antigens specifically expressed by that organ. Despite immunosuppressor treatments carried out, this reaction often results in rejection of the transplanted organ (this is the main cause of failure of allogeneic transplants). Apart from certain super-acute or acute rejections which involve essentially humoral responses, in the majority of cases, organ transplant rejection is essentially mediated by effector T lymphocytes. The present invention provides a novel approach to the prevention or treatment (e.g., the reduction or delay) of organ transplant rejection using Tregs specific for an antigen according to the invention effective to suppress or reduce the activity of such deleterious Teffs.

Accordingly, in particular embodiment, the method of preventing or treating an organ transplant rejection in a patient in need thereof comprises the following steps of:
a) isolating a population of Tregs from the patient;
b) obtaining in vitro or ex vivo a population of Tregs specific for an antigen which is not involved in the immune disease to be treated, by any of the methods described above;
c) administering to said patient in need thereof the population of step b).

In a particular embodiment, the method further comprises administering the antigen used in step b) to the patient. Said administration can be performed simultaneously, separately or sequentially by direct administration of the antigen to the patient. In a particular embodiment, the irrelevant exogenous antigen is selected from the group consisting of irrelevant non-pathogenic exogenous antigens (e.g. a food antigen from a common human diet including ovalbumin, fragments and variants thereof, or a protein from a non-related organism such as e.g., KLH) and irrelevant pathogenic antigens (e.g. allergens and alloantigens).

Typically, such Tregs are expanded and activated by culture in the presence of an antigen according to the invention. These cells may be produced for instance by culture in the presence of APCs that are autologous. These specific Tregs can then be injected to the patient, either before, together and/or after organ transplantation, thereby reducing the destructive activity of effector T cells.

The invention is also suited for the treatment of allergies, which are mediated by immune responses against particular antigens called allergens. By administering to the patients Tregs specific for an antigen according to the invention, it is possible to reduce these deleterious immune responses. Accordingly, in particular embodiment, the method of preventing or treating allergy in a patient in need thereof comprises the following steps of:
a) isolating a population of Tregs from the patient;
b) obtaining in vitro or ex vivo a population of Tregs specific for an antigen which is not involved in the immune disease to be treated, by any of the methods described above;
c) administering to said patient in need thereof the population of step b).

In a particular embodiment, the method further comprises administering the antigen used in step b) to the patient. Said administration can be performed simultaneously, separately or sequentially by direct administration of the antigen to the patient.

In a particular embodiment, the antigen is selected from the group consisting of irrelevant non-pathogenic exogenous antigens (e.g. a food antigen from a common human diet including ovalbumin, fragments and variants thereof or a protein from a non-related organism such as e.g., KLH) and irrelevant pathogenic antigens (e.g. alloantigens).

It should further be noted that for treating or allergies, Tregs are preferably isolated from the patient and activated by autologous APCs.

Various administration routes and protocols may be used to perform the present invention. These may be adapted by the skilled person, depending on the pathology to be treated. Generally, systemic or local administration(s) may be envisioned, such as intravenous, intraarterial, intraperitoneal, intramuscular, subcutaneous, etc. The cells (Tregs specific for an antigen of the invention and/or APCs pulsed with said antigen) may be injected during surgery or by any suitable means, such as using a syringe, for instance. For controlling diseases like GVHD or organ transplant rejection, the cell composition may be administered prior to, during or after transplantation. Furthermore, additional administrations of Tregs specific for an antigen of the invention and/or APCs pulsed with said antigen may be performed after transplantation, to further prevent or delay the immunopathology.

### Product

The present invention further relates to a product comprising:
a) an irrelevant antigen and
b) a population of Tregs activated by said antigen, as a combined preparation for simultaneous, separate or sequential use for preventing or treating an immune disease.

In one embodiment, the irrelevant antigen is an irrelevant non-pathogenic exogenous antigen (e.g. a food antigen from a common human diet including ovalbumin, fragments and variants thereof or a protein from a non-related organism such as e.g., KLH).

In another embodiment, the irrelevant antigen is an irrelevant pathogenic antigen (i.e. allergens, alloantigens).

In another particular embodiment, the irrelevant antigen may be formulated into a product consisting of a composition (pharmaceutical or not) comprising only the antigen with including eventually one or more pharmaceutically acceptable and/or approved carriers, additives, antibiotics, preservatives, adjuvants, diluents and/or stabilizers.

In another particular embodiment, the irrelevant antigen may be formulated into a product consisting of pharmaceutical composition comprising antigen presenting cells (APCs) pulsed with said antigen, a composition including eventually one or more pharmaceutically acceptable and/or approved carriers, additives, antibiotics, preservatives, adjuvants, diluents and/or stabilizers.

The invention will be further illustrated by the following examples. However, these examples and figures should not be interpreted in any way as limiting the scope of the invention.

### DESCRIPTION OF THE FIGURES

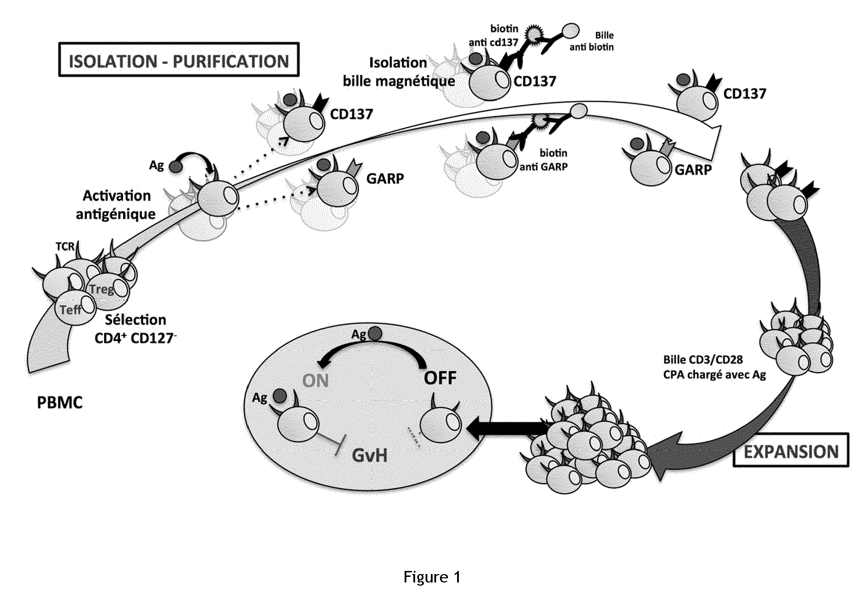

**Figure 1****:** Principal diagram of the method of the invention.

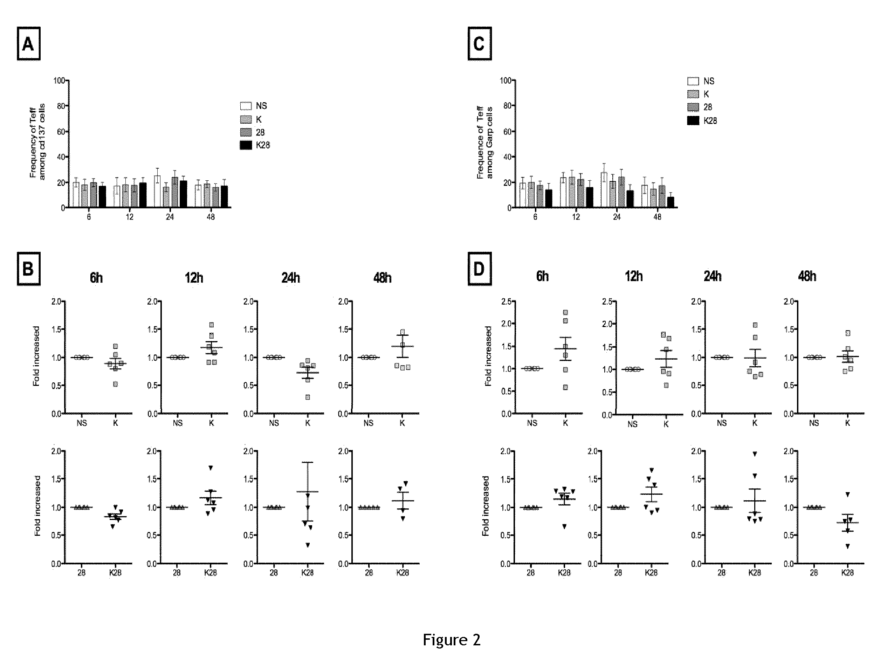

**Figure 2****:** Teffs frequency among cells expressing CD137 or GARP: 2 million Peripheral Blood Mononuclear Cells (PBMC) have been stimulated with medium (NS) or 100µg/ml of KLH(K) or 1µg/ml of anti-CD28 antibody (28) or with KLH and anti-CD28 antibody (K28) during 6, 12, 24 and 48 hours at 37°C. After this stimulation (activation), these cells have been labeled with fluorochrome conjugated antibodies directed to CD3, CD4, CD25, FOXP3 and CD137 (A and B) or GARP (B and D), then Identified by flow cytometry (n=5). Teffs frequency among CD137⁺ cells (A) or GARP⁺ cells (C). Standardized frequency versus medium (Top) or versus 28 (Bottom) (B and D), p<0.05 (Anova).

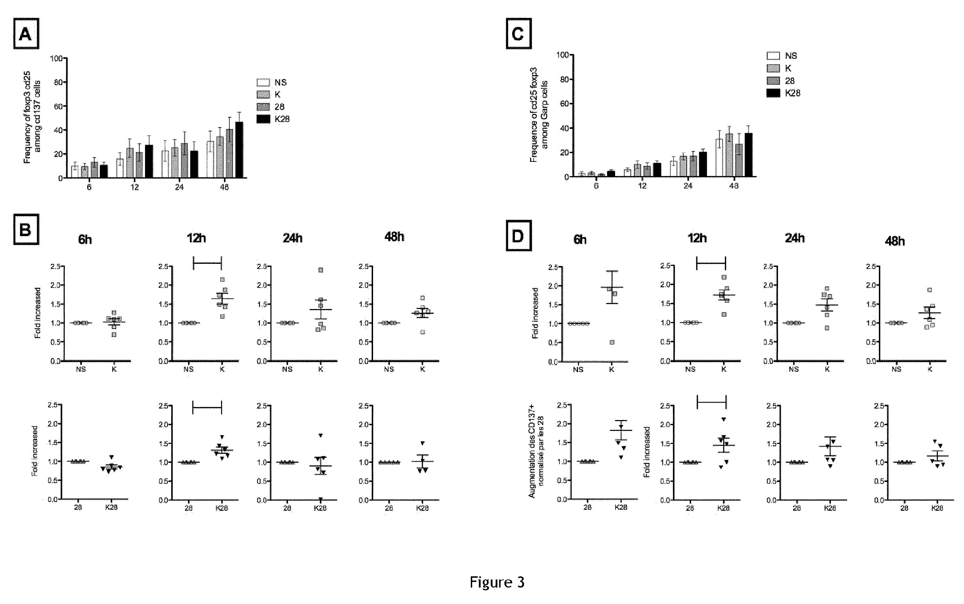

**Figure 3****:** Tregs frequency among CD137⁺ (A, B) or GARP⁺ cells (C, D). Stimulation and labelling have been performed as described in figure 2. Tregs frequency among CD137⁺ cells (A) or GARP⁺ cells (C). Standardized frequency versus medium (Top) or versus 28 (Bottom) (B and D)), p<0.05 (Anova).

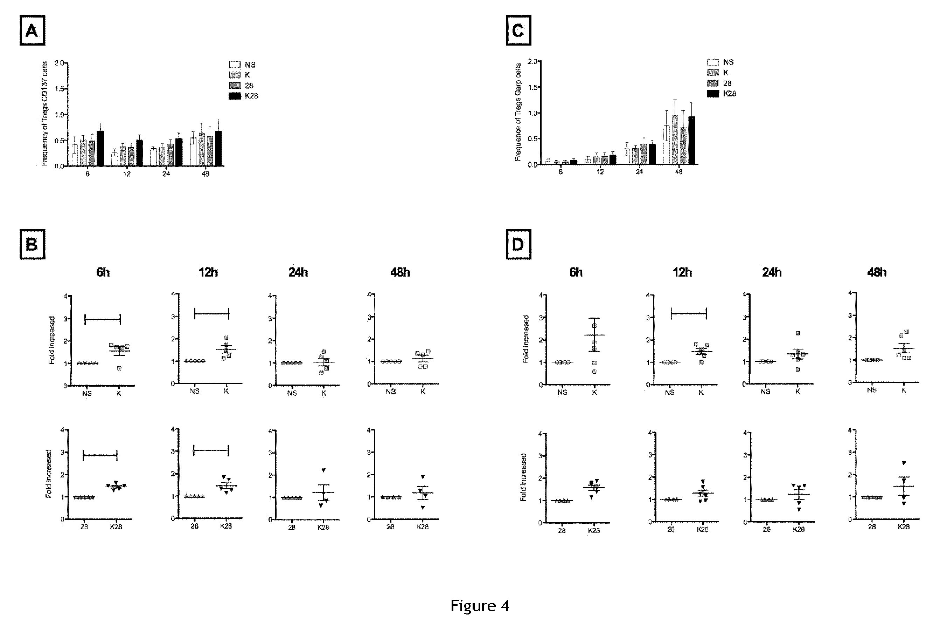

**Figure 4****:** CD137⁺ Tregs frequency (A, B)or GARP⁺ Tregs frequency (C, D) among PBMC. Stimulation and labelling have been performed as described in figure 2. CD4⁺CD25^{high}FOXP3^{high}CD137⁺ frequency (A) or CD4⁺CD25^{high}FOXP3^{high}GARP⁺ (C). Standardized frequency versus medium (Top) or versus 28 (Bottom) (B and D), p<0.05 (Anova).

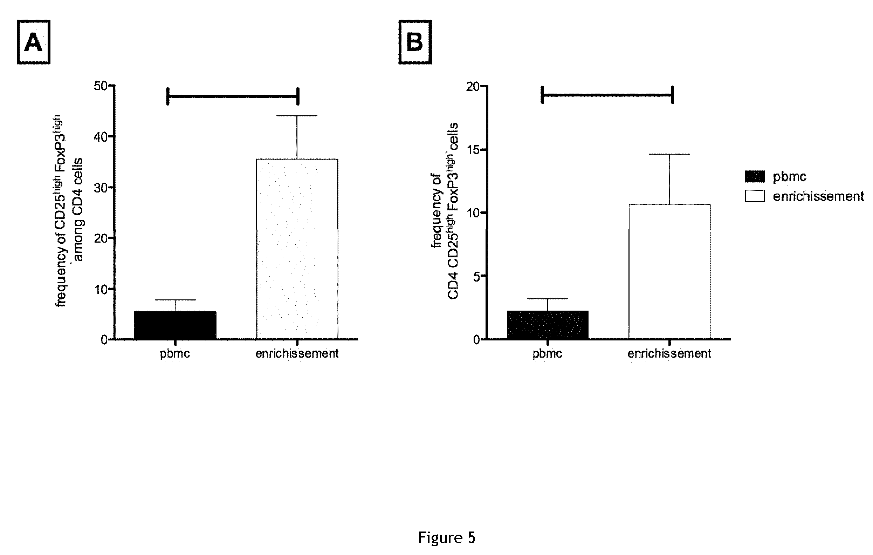

**Figure 5****:** Tregs CD4⁺CD25^{high}FOXP3^{high} frequency amongst CD4⁺ cells (A) or PBMC (B) before and after enrichment step.

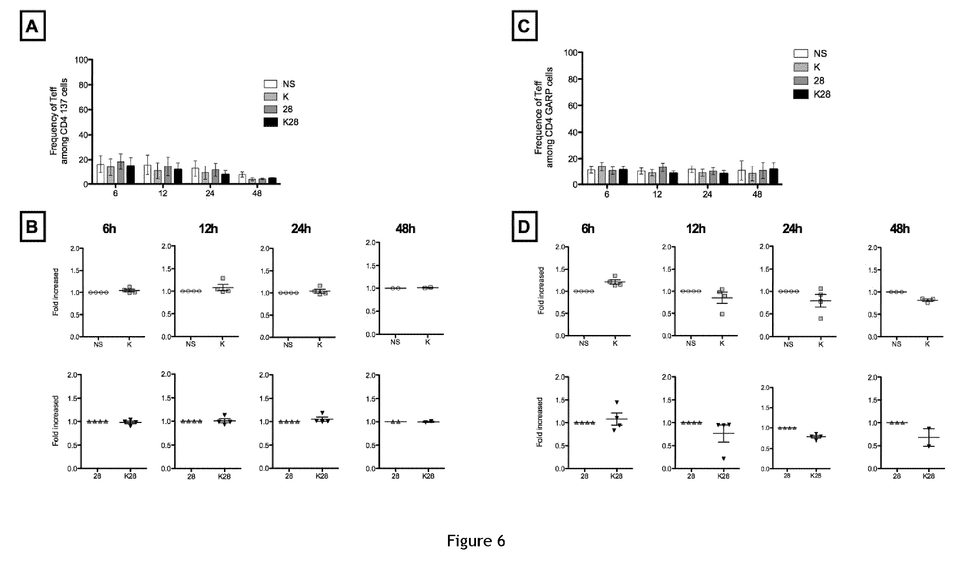

**Figure 6****:** Teffs frequency among CD137⁺ cells or GARP⁺ cells after antigenic stimulation of CD4⁺CD127⁻ cells. After the enrichment step, 2 million of CD4⁺CD127⁻ cells have been stimulated and labeled as mentioned in figure 2 (n=4). Teffs frequency among CD137⁺ cells (A) or GARP+ cells (C). Teffs frequency among CD137⁺ cells (B) or GARP⁺ cells (D) standardized frequency versus medium (Top) or versus 28 (Bottom), p<0.05 (Anova).

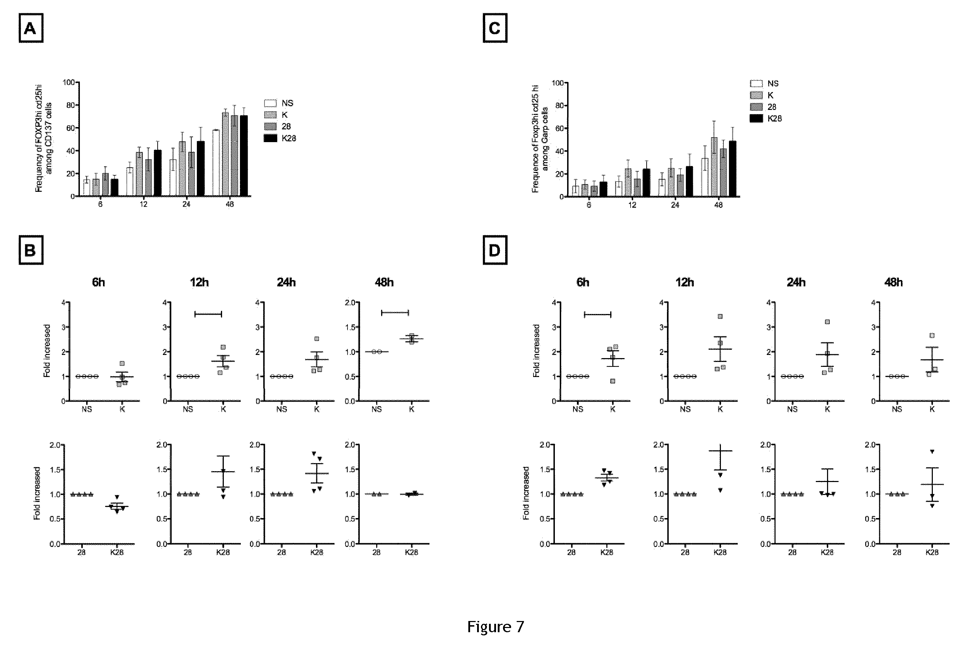

**Figure 7****:** Tregs frequency among CD137⁺ (A, B) or GARP⁺ cells (C, D) after antigenic stimulation of CD4⁺CD127⁻ cells. After the enrichment step, 2 million of CD4⁺CD127⁻ cells have been stimulated and labeled as mentioned in figure 2 (n=4). Tregs frequency among CD137⁺ cells (A) or GARP⁺ cells (C). Tregs frequency among CD137⁺ cells (B) or GARP⁺ cells (D) standardized frequency versus medium (Top) or versus 28 (Bottom), p<0.05 (Anova).

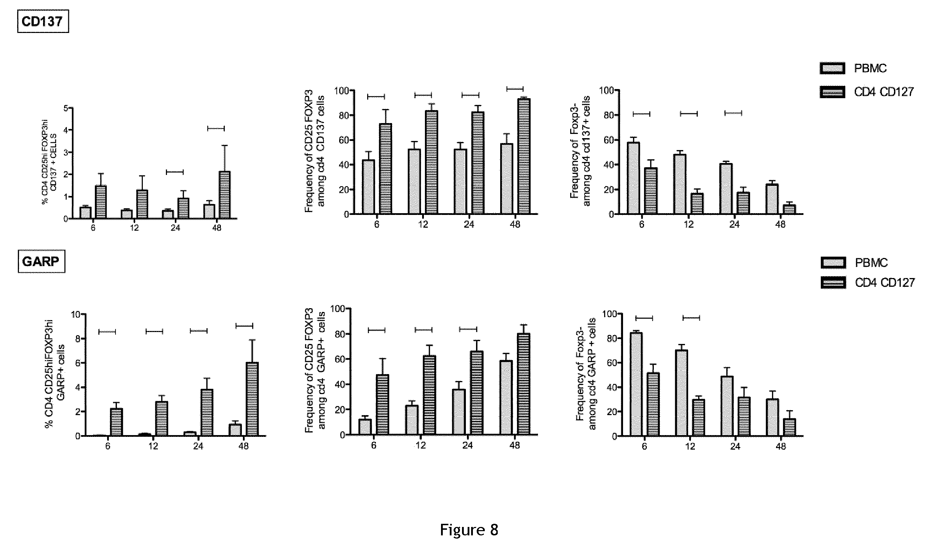

**Figure 8****:** Teffs contamination and Tregs frequency of PBMC stimulated with KLH before and after enrichment step among CD137⁺ cells (Top) or GARP⁺ cells (Bottom).

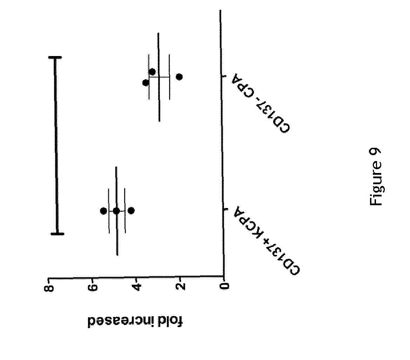

**Figure 9****:** Tregs count after expansion step. After enrichment step, CD4⁺ cells have been stimulated with Antigen Presenting Cells (APC) (CD3⁻) during 16h with 100µg KLH. CD137⁺ and CD137⁻. Tregs have been isolated by flow cytometry and cultivated with APC or APC+KLH, respectively during 10 days. Culture medium has been changed every day. Expansion ratio has been determined by dividing cell number counted after 10 day by starting cell number.

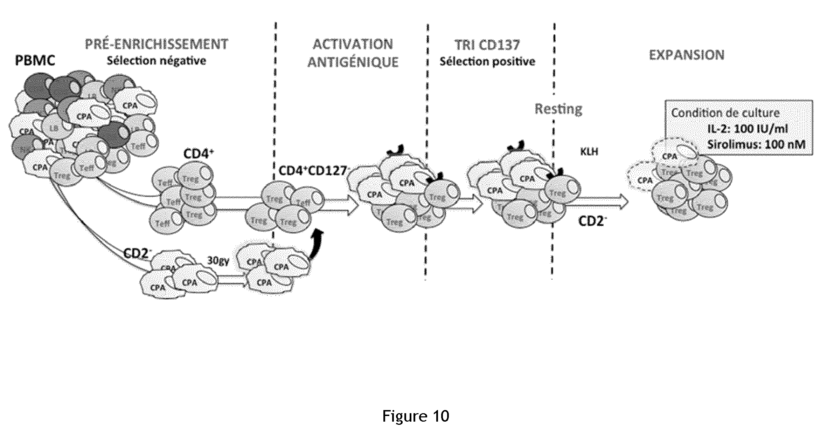

**Figure 10****: Principal diagram of the method of invention using irradiated APCs.**

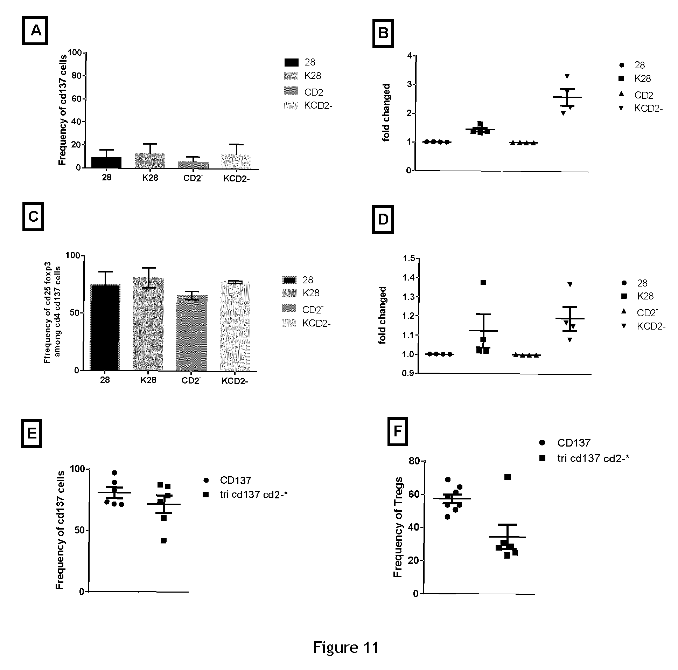

**Figure 11****: Treg frequency with or without irradiated APCs.** Tregs were enriched from PBMC using a first negative selection of CD4+ cells followed by a second negative selection of CD4+ CD127-. One million of CD4+ CD127- cells were stimulated 12h at 37°C with either 1µg/ml anti-CD28 antibody (28); anti-CD28 antibody and KLH (K28); 1 million of CD2- cells irradiated at 30 gy (CD2-); or with KLH and irradiated CD2- cells (KCD2-). After this stimulation, CD4+ CD127- cells have been labeled with fluorochrome conjugated antibodies directed to CD3, CD4, CD25, FoxP3 and CD137 and identified by flow cytometry (A, B, C, D, n= 4). CD137+ cells frequency (A) and Tregs frequency among CD137+ cells (C) are shown. B and D show respectively fold change of CD137+ and Treg before and after stimulation.
   After stimulation, CD4+ CD127- cells are incubated with anti-CD137 biotinylated antibody then with anti-biotin antibody coupled to magnetic beads. CD137+ cells retained by a column under magnetic field are then eluted and characterized by flow cytometry (E, F). Frequency of CD137+ cells (E) and Treg cells (F) are shown (n=6).

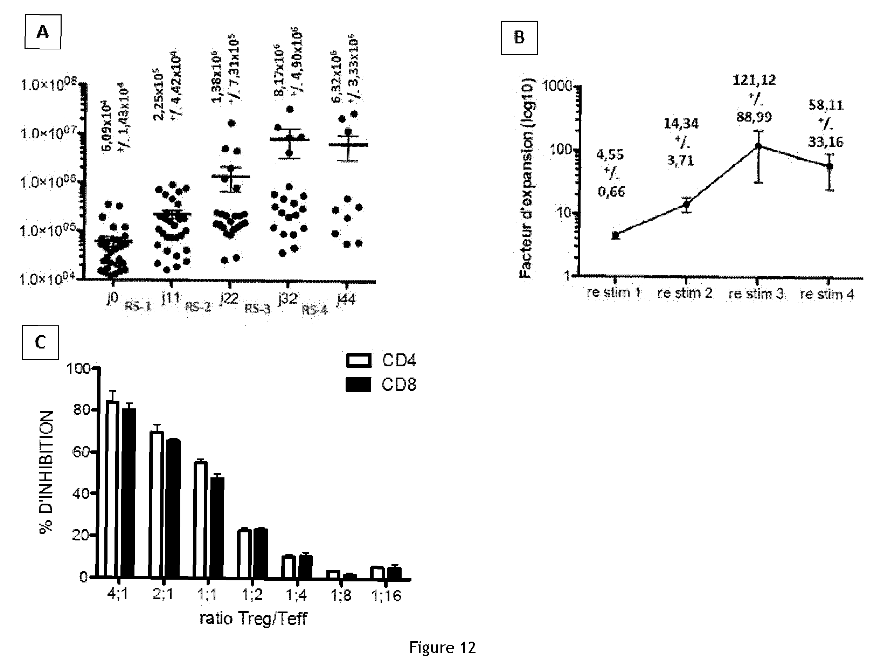

**Figure 12****. Expansion of Exo-Tregs using irradiated APCs.** CD4+ CD127- cells were selected from 4x10⁸ PBMC. These cells were stimulated by KLH and irradiated CD2-(ratio 1:1) for 12h at 37°C. CD137+ cells were then selected and cultivated with RPMI medium containing 10%FCS, 1%PS, 2.5% Hepes 100U/ml IL2 and 100nM rapamycin (sirolimus) for 24 to 48h. Cells were then restimulated 3 to 4 times by adding KLH-loaded irradiated APCs (ratio 1:2). Number of cells (A) and expansion factor (B) were determined before each stimulation (n=35). (C) At day 32, frequency of proliferation inhibition of CD4 and CD8 wells was determined by flow cytometry at different ratios of Treg/Teff cells after 5 days of culture.

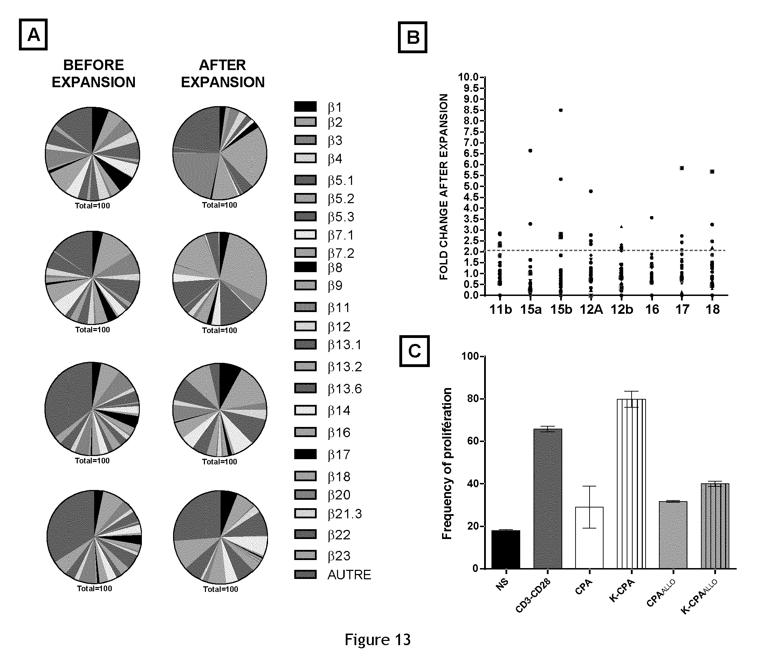

**Figure 13****. In vitro exo-Tregs specificity after expansion.** After enrichment and expansion, antigen specificity of KLH-Tregs was determined in vitro. (A) T-cell receptor Vbeta repertoire before and after 32 days of expansion (B) Dot plot showing fold change of TCR Vbeta expression after 32 days of expansion (frequency of each Vbeta after expansion are normalized to frequency before expansion) (n=8). (C) After expansion, cells have been labeled with proliferation marker coupled with allophycocyanin followed by CD4 selection and culture with autologous or allogenic (ALLO) irradiated CD2- cells with ou without KLH. After 5 days of culture, allophycocyanin dilution was determined using flow cytometry.

### EXAMPLES

### Methods and materials

### Results

### Evaluating Tregs specificity and Teffs proportion in CD137 and GARP positive cells selected from PBMC population after antigenic activation with KLH without Performing enrichment step.

Firstly, it was tested whether an antigenic stimulation is capable to modify ratio between Teffs (CD4⁺FOXP3⁻ cells) and Tregs (CD4⁺CD25^{high}FOXP3^{high} cells) among CD137 positive or GARP positive cells without performing enrichment step.

2 million of PBMC were cultured in RPMI medium 10% of bovine fetal serum without antigenic stimulation (NS) or stimulated with 100µg/ml of KLH(K) or 1µg/ml of anti-CD28 antibody (28) or with KLH and anti-CD28 antibody (K28) during 6, 12, 24 and 48 hours at 37°C. After the activation, these cells have been labeled with fluorochrome conjugated antibodies directed to CD3, CD4, CD25, FOXP3 and CD137 (Figure 2A and B) or GARP (Figure 2B and D), then identified by flow cytometry (n=5).

Since the frequency of antigen KLH specific cells was low, the different frequencies obtained in plates stimulated with KLH were standardized with their control, respectively (KLH vs NS and K28 vs 28). The data were indicated on figure 2B and 2D. These results show that independently of stimulation way, KLH does not induce significant modification of Teffs frequency in CD137 cells (figure 2A and 2B) or GARP cells (figure 2C and 2D). These results also suggest that selecting CD137⁺ or GARP⁺ cells conduct to Teffs contamination respectively of 17.75% ± 4.05 and 19.86% ± 5.22.

Regarding Tregs CD4⁺ CD25^{high}FOXP3^{high} cells frequency in CD137 or GARP positive cells, there is no significant difference between different stimulations (Figure 3A and 3C). However, after standardization, it was observed that Tregs number significantly increases among CD137⁺ and GARP⁺ cells after 12 hours of stimulation with KLH with or without CD28 suggesting a specific response to KLH. These results indicate that stimulating PBMC population, then identifying activated Tregs in CD137 or GARP positive cells after 12 hours of stimulation allow obtaining cell population containing 24.8±7.6% (CD137) and 10.4±3.1% (GARP) Tregs respectively.

After that, it was determined whether PBMC stimulation allows enriching the cell population of CD137⁺ Tregs or GARP⁺ Tregs. Figures 4A and 4B (CD4⁺CD25^{high} FOXP3^{high}CD137⁺) or Figure 4C and 4D (CD4⁺CD25^{high}FOXP3^{high}GARP⁺) show that frequency of these populations is low during 48 h. CD137⁺ Tregs and GARP⁺ Tregs are 0.37% and 0.14%, respectively from PBMC population 12 hours after their stimulation. However, after 12 hours of stimulation the number of CD137⁺ and GARP⁺ was increased.

These results indicate that it is possible to isolate Tregs specific to irrelevant antigen by selecting CD137⁺ and GARP⁺cells from PBMC population after 12 hours of antigenic stimulation. However, the number of Tregs is low (24.8±7.6% and 10.4±3.1%) and the number of Teffs is high (17.75±4.05% and 19.86±5.22%).

Thus, it was demonstrated that antigenic stimulation of PBMC in the aim to obtain specific Tregs for an irrelevant antigen by CD137⁺ and GARP⁺ positive cell selection is possible but the number of Tregs is low and the number of Teffs is still high. Consequently, such Tregs population is not suitable for use in medical environment.

### Evaluating specific Tregs and Teffs proportions among CD137 and GARP positive cells selected from PBMC population after antigenic activation with KLH after performing enrichment step.

In the second time, it was evaluated whether enriching PBMC population with Tregs previously to the antigenic stimulation allow increasing Tregs activated frequency and decreasing Teffs contamination.

The previous enrichment of Tregs was performed by adding to PBMC population the "Miltenyi® enrichment cocktail" which contains anti-CD14, anti-CD8, anti-CD19, anti-CD36, anti CD56, anti-CD123 and anti TCRγ/δ biotin-conjugated antibodies allowing selecting CD4⁺ cells and anti-CD127 antibodies allowing selecting CD127⁻ cells.

After this negative selection (CD4⁺ and CD127⁻), immunolabeling was performed in order to determine by flow cytometry Tregs (defined as CD4⁺CD25^{high}FOXP3^{high} cells) frequency.

As shown on Figure 5, the proportion of Tregs population was increased (from 2.28±0.5% to 10.67±1.98% of total cell population). Moreover, among CD4⁺ cells, 35.5±4.2% Tregs were observed after enrichment.

Following enrichment step, the Tregs enriched population was stimulated with KLH antigen.

Figure 6 shows that Teffs contamination following CD137 or GARP cells selection was 11.05±1.13% and 10.45±0.41% respectively. Thus, it appears that Teffs contamination was decreased two fold after the enrichment step.

On the other hand, figure 7 shows that Tregs proportion was significantly increased in CD137⁺ or GARP⁺ cells after 6 or 12 hours of KLH stimulation. These results indicate that selecting CD4⁺CD127⁻ cells and CD137 and GARP positive cells after 12h or 6 h of antigenic stimulation, respectively allow obtaining Tregs cell population containing 35.45±4.54% (CD137) and 10.84±3.35% (GARP) of Tregs.

These results were confirmed by several trials comparing proportion of activated Tregs expressing CD137 or GARP obtained by PBMC population stimulated before or after an enrichment step.

Thus, figure 8 clearly shows that performing an enrichment step by CD4⁺CD127⁻ selection (two negative selection) from PBMC population followed by selecting CD137 and GARP positive cells allow obtaining a cell population characterized by low contamination of Teffs and increased frequency of activated Tregs.

### Irradiation of APC limits contamination by CD137⁺ cells

The method described above (hereafter, the "first method") enables the isolation of a CD137 positive fraction containing Tregs. However, said fraction also comprises some CD137⁺ APCs. After 22 days of expansion, KLH-specific Tregs were obtained but some contamination with B lymphocytes and monocytes remained. Thus the expansion step does not lead to the total disappearance of the initial contamination by other CD137⁺ cells.

An improved method (hereafter, the "second method") was therefore designed in order to limit contamination by these CD137⁺ cells **(****Figure 10****).** Tregs are enriched from PBMC using a first negative selection of CD4⁺ cells followed by a second negative selection of CD4⁺ CD127⁻. CD4⁺ CD127⁻ cells are stimulated with KLH and irradiated CD2⁻ cells. After antigenic activation, CD137⁺ cells are selected, rested and then are expanded.

In order to compare the two methods, the frequency of Tregs (CD25^{high}Foxp3^{high}) amongst CD137⁺ cells after antigenic activation was evaluated with the first (activation KLH/CD28 on pre-enriched cells) and the second method wherein irradiated CD2⁻ cells were added. **Figure 11A and 11C** show that addition of irradiated CD2⁻ cells allows selection of KLH-specific Tregs and that this stimulation is more specific and reproducible. The magnitude difference between with and without KLH is higher with irradiated CD2⁻ APCs than with the first method **(****Figure 11D****)**

After antigenic activation, of CD137⁺ cells were sorted and CD137⁺ cells and the frequency of Tregs was determined within the CD137 positive fraction **(****Figure 11E and 11F****).** Frequency of CD137⁺ cells is not different between the two methods **(****Figure 11E****)** whereas Tregs frequency is lower with irradiated CD2⁻ cells **(****Figure 11F****).** This difference of Tregs frequency is directly due to addition of CD2⁻ cells.

Therefore, the second method allows selection and purification of KLH-specific Tregs. Expansion of these cells was consequently performed from 35 donor samples following the protocol outlined in **Figure 10**.

Results are shown in **Figure 12****.** After 3 round of re-stimulation, a decline in cell number and expansion factor was observed. This indicates that KLH-Tregs should be used before the 4^{th} re-stimulation for in vivo experiments. A high variability was detected during expansion between the different donor samples.

In order to assay whether the Tregs thus obtained retain their immunosuppressive function, inhibition of autologous Teff was studied. After 32 days of expansion, Tregs are still capable of inhibiting proliferation of autologous CD4 and CD4 T lymphocytes **(****Figure 12C****).**

The T-cell receptor Vbeta repertoire of the KLH-Tregs thus obtained was analyzed **(****Figure 13A and 13B****).** After 32 days of expansion, an enrichment of particular Vbeta was observed **(****Figure 13A****).** Specifically, expression of at least 3 Vbeta was more than doubled for each donor sample **(****Figure 13B****).** As expected, the amplified Vbeta vary from one donor to another.

The specificity of the Treg obtained was tested by analysing the proliferation of the Tregs in response to KLH **(****Figure 13C****).** In presence of KLH, a proliferation comparable to a polyclonal stimulation was observed. These results suggest that we were able to select and enrich KLH-specific Tregs.

### REFERENCES

Bacher & Scheffold, Cytometry A, 2013 Aug;83(8):692-701. doi: 10.1002/cyto.a.22317
Bacher et al., Mucosal Immunol, 2014 Jul;7(4):916-28. doi: 10.1038/mi.2013.107
Battaglia & Rancarolo, Eur J Immunol. 2009 Dec;39(12):3296-300. doi: 10.1002/eji.200940117
Brunstein, C.G. et al. Infusion of ex vivo expanded T regulatory cells in adults transplanted with umbilical cord blood: safety profile and detection kinetics. Blood (2011).
Cohen, J. L., Trenado, A., Vasey, D., Klatzmann, D. & Salomon, B .L . CD4(+)CD25(+) immunoregulatory T Cells: new therapeutics for graft- versus-host disease. J Exp Med 196, 401-6. (2002).
Di lanni, M. et al. Tregs prevent GVHD and promote immune reconstitution in HL haploidentical transplantation. Blood 117, 3921-8 (2011).
Ferrara, J.L., Levine, J.E., Reddy, P. & Holler, E. Graft-versus-host disease. Lancet 373, 1550-61 (2009).
Noyan et al., Eur. J. Immunol. 2014. 44 : 2592-2602. doi: 10.1002/eji.201344381
Sagoo, P. et al. Human regulatory T cells with alloantigen specificity are more potent inhibitors of alloimmune skin graft damage than polyclonal regulatory T cells. Sci Transl Med 3, 83ra42 (2011).
Tang, Q. et al. In vitro-expanded antigen-specific regulatory T cells suppress autoimmune diabetes. J Exp Med 199, 1455-65 (2004).
Trenado, A. et al. Recipient-type specific CD4+CD25+ regulatory T cells favor immune reconstitution and control graft-versus-host disease while maintaining graft-versus-leukemia. J Clin Invest 112, 1688-96 (2003).

## Claims

1. An *in vitro* or *ex vivo* method for obtaining a population of regulatory T cells (Tregs) specific for an irrelevant antigen from a PBMC population, comprising the steps of:
a) obtaining CD4⁺ cells from said PBMC population by negative selection;
b) contacting the CD4⁺ cells of step a) with an irrelevant antigen;
c) selecting the cells expressing a marker specific of activated Tregs from the population obtained in step b), said marker being selected from the group consisting of CD137 and GARP); and
d) expanding the activated Tregs of step c).

2. The method of claim 1, wherein step a) comprises the further steps of:
i) adding antibodies directed against-CD14, CD8, CD19, CD36, CD56, CD123, and TCRγ/∂ to said PBMC population; and
ii) recovering the cells not bound by any antibody in step i).

3. The method of claim 2, further comprising adding an antibody directed against CD127 and recovering the cells not bound by said antibody.

4. The method of claim 3, wherein said antibody directed against CD127 is added to the PBMC population of step i) or to the cells recovered in step ii).

5. The method of any one of the previous claims, wherein said irrelevant antigen is selected from non-pathogenic exogenous antigen or irrelevant pathogenic antigen, preferably said irrelevant antigen is Keyhole Limpet Hemocyanin (KLH).

6. The method of any one of the previous claims, wherein the antigen of step b) is presented by antigen-presenting cells (APCs).

7. The method of any one of the previous claims, wherein step d) comprises growing said Tregs in the presence of APC presenting said irrelevant antigen.

8. The method any one of the previous claims, wherein the APCs have been previously irradiated.

9. The method of claim 7, wherein said Tregs are grown in step d) for at least 2 generation times.

10. The method of any one of the previous claims, wherein selection is performed by magnetic activated cells sorting.

11. A population of Tregs obtained by the method of any one of claims 1 to 10.

12. A pharmaceutical composition comprising at least one population of Tregs of claim 11 with one or more pharmaceutically acceptable carrier.

13. The population of Tregs of claim 11 for use as a drug.

14. The population of Tregs according to claim 11 for use in the treatment or the prevention of a disease caused by pathological T cells.

15. The population of Tregs for use according to claim 14, wherein said use further comprises the administration to said patient of the irrelevant antigen used in step b), simultaneously, separately or sequentially.

16. A product comprising a) an irrelevant antigen and b) the population of T cells activated by said antigen of claim 11, as a combined preparation for simultaneous, separate or sequential use for preventing or treating disease caused by pathological T cells.
